# EUROPEAN PATENT APPLICATION

(11) **EP 2 130 480 A1**
(43) Date of publication of application: **09.12.2009**
(21) Application number: 08739343.5
(22) Date of filing: 28.03.2008
(51) Int. Cl.: A61B 1/00, A61B 5/07

(54) **CAPSULE TYPE MEDICAL DEVICE AND METHOD FOR PRODUCING CAPSULE TYPE MEDICAL DEVICE**

(30) Priority: 30.03.2007 JP 2007094892
(71) Applicant: Olympus Medical Systems Corp., Tokyo 151-0072 (JP); Olympus Corporation, Tokyo 151-0072 (JP)
(72) Inventor: SEGAWA, Hidetake, Tokyo 151-0072 (JP); FUJIMORI, Noriyuki, Tokyo 151-0072 (JP)
(74) Representative: von Hellfeld, Axel
(86) International application number: PCT/JP2008/056225
(87) International publication number: WO 2008/123464

(57) **Abstract**

An object of the present invention is that a capsule medical device can be manufactured, even if a part of functional components mounted on circuit boards is in a failed state, without wastefully discarding the remaining functional components in a good product state. According to a manufacturing method of a capsule medical device according to the present invention, light-emitting elements 3a to 3d and a solid-state imaging device 5 are mounted on a series of flexible boards 20a having a series of flexible circuit board structure, light-emitting elements 6a to 6d, a solid-state imaging device 8, and a wireless unit 9a are mounted on a series of flexible boards 20b, which is a separate body from the series of flexible boards 20a, and the series of flexible boards 20a and 20b are board-to-board connected to a control board 19c, which is a separate body from the series of flexible boards 20a and 20b.

## Description

### TECHNICAL FIELD

The present invention relates to a capsule medical device introduced into internal organs of a subject such as a patient to acquire in-vivo information of the subject, and a method of manufacturing a capsule medical device.

### BACKGROUND ART

Conventionally, in the field of endoscope, a swallowing-type capsule endoscope having an imaging function and a wireless communication function has been proposed. The capsule endoscope is introduced into internal organs by swallowing it from a mouth of the subject such as a patient for observation (examination) of the internal organs. Thereafter, the capsule endoscope moves in the internal organs with peristaltic movements or the like, while sequentially capturing images of inside of the subject (hereinafter, occasionally "in-vivo images") at a predetermined interval, for example, at an interval of 0.5 second, and finally, it is naturally discharged to the outside of the subject.

The in-vivo images captured by the capsule endoscope while the capsule endoscope is present inside the internal organs of the subject are sequentially transmitted from the capsule endoscope to an external receiving device by wireless communication. The receiving device is carried by the subject to receive an in-vivo image group wirelessly transmitted from the capsule endoscope introduced into the internal organs of the subject, and stores the received in-vivo image group on a recording medium.

The in-vivo image group stored on the recording medium of the receiving device is taken in an image display device such as a workstation. The image display device displays the in-vivo image group of the subject acquired via the recording medium. A doctor, a nurse or the like can diagnose the subject by observing the in-vivo image group displayed on the image display device.

The capsule endoscope has a capsule casing with a transparent optical dome at an end, and includes, inside the capsule casing, an illuminating unit such as an LED that illuminates inside of the internal organs over the optical dome, an optical unit such as a lens that forms images of the inside of the internal organs illuminated by the illuminating unit, and an imaging unit such as a CCD that captures images of the inside of the internal organs (that is, in-vivo image) formed by the optical unit (for example, see Patent Documents 1 and 2). Further, as the capsule endoscope, there is a binocular-lens capsule endoscope having optical domes at forward-side end and backward-side end of a capsule casing, and including a forward-side imaging mechanism that captures images of inside of the internal organs over the forward-side optical dome (forward-side in-vivo images), and a backward-side imaging mechanism that captures images of the inside of the internal organs over the back-side optical dome (back-side in-vivo images) in the capsule casing. Each of the forward-side and back-side imaging mechanisms includes an illuminating unit that illuminates the inside of the internal organs over the optical dome, an optical unit that forms images of the inside of the internal organs illuminated by the illuminating unit, and an imaging unit that captures images of the inside of the internal organs formed by the optical unit.

Patent Document 1: Japanese Patent Application Laid-open No. 2005-198964
Patent Document 2: Japanese Patent Application Laid-open No. 2005-204924

### DISCLOSURE OF INVENTION

### PROBLEM TO BE SOLVED BY THE INVENTION

When a conventional binocular-lens capsule endoscope is manufactured, a rigid flexible board, on which the forward-side and back-side imaging mechanisms, and a wireless communication unit and the like are mounted, is arranged inside the capsule casing. The rigid flexible board has a series of board structure in which a rigid circuit board (hereinafter, simply "rigid board") such as an illuminating board, an imaging board, or a wireless board and a flexible circuit board (hereinafter, simply "flexible board") for connecting between a required number of rigid boards are integrally formed. The illuminating unit and the imaging unit of the forward-side imaging mechanism are mounted on the illuminating board and the imaging board, respectively, arranged on the forward side inside the capsule casing, among the series of rigid boards forming the rigid flexible board, and the illuminating unit and the imaging unit of the back-side imaging mechanism are mounted on the illuminating board and the imaging board, respectively, arranged on the back side in the capsule casing. Further, the optical unit in the forward-side imaging mechanism is fitted to the illuminating board and the imaging board on the forward side, and the optical unit in the back-side imaging mechanism is fitted to the illuminating board and the imaging board on the back side.

In the conventional binocular-lens capsule endoscope manufactured by arranging such a rigid flexible board inside the capsule casing, if a failure occurs in a part of various parts (for example, the forward-side and back-side imaging mechanisms) mounted on the rigid flexible board, all parts mounted on the rigid flexible board need to be replaced together with the rigid flexible board, even if the remaining parts operate normally (in a good product state). Specifically, if a failure such as defective assembly occurs in one of the forward-side and back-side imaging mechanisms mounted on the rigid flexible board, even if the other imaging mechanism is in a good product state, these imaging mechanisms need to be replaced together with the rigid flexible board, and the other imaging mechanism in a good product state needs to be discarded wastefully, together with the imaging mechanism in a failed state.

The present invention has been made in view of such circumstances, and an object of the present invention is to provide a capsule medical device that can be manufactured, even if a part of functional components mounted on a circuit board is in a failed state, without wastefully discarding remaining functional components in a good product state, and a method of manufacturing a capsule medical device.

### MEANS FOR SOLVING PROBLEM

To solve the problem described above and achieve the object, a method of manufacturing a capsule medical device according to the present invention includes a mounting step of mounting one or more functional components on each of a first circuit board group and a second circuit board group, which are separate bodies from each other, and mounting a control unit that controls an operation of the one or more functional components, on a control board that is a separate body from the first circuit board group and the second circuit board group; and a board connecting step of connecting the first circuit board group and the second circuit board group to the control board.

The method of manufacturing a capsule medical device according to the present invention further includes a verifying step of verifying whether the one or more functional components mounted on the first circuit board group operate normally, verifying whether the one or more functional components mounted on the second circuit board group operate normally, and verifying whether the control unit mounted on the control board operates normally. At the board connecting step, the first circuit board group in a good product state and the second circuit board group in a good product state having been determined to operate normally at the verifying step are connected to the control board in a good product state having been determined to operate normally at the verifying step.

In the method of manufacturing a capsule medical device according to the present invention, at the mounting step, the one or more functional components are mounted on same side surfaces of boards of the first circuit board group, and the one or more functional components are mounted on same side surfaces of boards of the second circuit board group.

The method of manufacturing a capsule medical device according to the present invention further includes a board forming step of separately forming the first circuit board group, which is an integrally formed flexible circuit board including an illuminating board and an imaging board, the second circuit board group, which is an integrally formed flexible circuit board including at least an illuminating board and an imaging board, and the control board, which is a rigid circuit board. At the mounting step, an illuminating unit and an imaging unit as functional components for capturing an in-vivo image of inside a subject are mounted on the illuminating board and the imaging board, respectively, in the first circuit board group, and an illuminating unit and an imaging unit as functional components for capturing an in-vivo image captured in a different direction than the in-vivo image are mounted on the illuminating board and the imaging board, respectively, in the second circuit board group.

In the method of manufacturing a capsule medical device according to the present invention, at the board forming step, the second circuit board group, which is a integrally formed flexible circuit board including the illuminating board, the imaging board, and a wireless board, is formed. At the mounting step, a wireless unit, which is a functional component for wirelessly transmitting the in-vivo images and the in-vivo images captured in the different direction to outside, is mounted on the wireless board in the second circuit board group.

The method of manufacturing a capsule medical device according to the present invention further includes a board arranging step of arranging circuit boards in a series of circuit boards formed of the first circuit board group, the second circuit board group, and the control board connected at the board connecting step, substantially parallel to each other and facing each other; and an inside-casing arranging step of arranging at least the series of circuit boards inside a capsule casing.

A capsule medical device according to the present invention includes a first circuit board group on which one or more functional components are mounted; a second circuit board group on which one or more functional components are mounted; and a control board on which a control unit that controls operations of the one or more functional components in the first circuit board group and the one or more functional components in the second circuit board group are mounted. The first circuit board group, the second circuit board group, and the control board are separate bodies from each other, and the first circuit board group, the second circuit board group, and the control board are formed as a series of circuit boards obtained by connecting good circuit boards each having been determined to operate normally to each other.

In the capsule medical device according to the present invention, the one or more functional components mounted on the first circuit board group and the one or more functional components mounted on the second circuit board group include functional components having a same function.

In the capsule medical device according to the present invention, the same function means an illuminating unit and an imaging unit for capturing an in-vivo image of inside a subject.

In the capsule medical device according to the present invention, the illuminating unit and the imaging unit mounted on the first circuit board group and the illuminating unit and the imaging unit mounted on the second circuit board group capture in-vivo images in directions different from each other.

### EFFECT OF THE INVENTION

In the manufacturing method of the capsule medical device according to the present invention, one or more functional components are mounted on each of a first circuit board group and a second circuit board group formed separately from each other, and the first circuit board group and the second circuit board group, on which required functional components are mounted, are connected to a control board, thereby manufacturing a series of circuit boards having the required functional components. Therefore, there is an effect such that, even if a part of the functional components mounted on the circuit board is in a failed state, only the functional component in the failed state can be replaced by a functional component in a good product state to manufacture the capsule medical device, without wastefully discarding the remaining functional components, which are in a good product state.

### BRIEF DESCRIPTION OF DRAWINGS

FIG. 1 is a schematic longitudinal cross section of a configuration example of a capsule endoscope according to an embodiment of the present invention.
FIG. 2 is a schematic diagram for exemplifying an internal structure of the capsule endoscope as viewed over an optical dome from a direction F shown in FIG. 1.
FIG. 3 is a schematic diagram for exemplifying the internal structure of the capsule endoscope as viewed over the optical dome from a direction B shown in FIG. 1.
FIG. 4 is a schematic diagram for exemplifying a state where circuit components of a power supply system are mounted on a control board.
FIG. 5 is a schematic diagram for exemplifying a state where a series of circuit boards folded and arranged in a casing of the capsule endoscope is developed.
FIG. 6 is a schematic diagram for explaining a manufacturing method of a series of circuit boards incorporated in a functional unit of the capsule endoscope.
FIG. 7 is a schematic diagram for exemplifying a state where a series of flexible boards are connected to a control board.
FIG. 8 is a schematic diagram for exemplifying a state where the series of flexible boards and the control board are board-to-board connected by using an anisotropic conductive adhesive.
FIG. 9 is a schematic diagram for exemplifying a state where the series of flexible boards and the control board are board-to-board connected by using a metal bump and an insulating adhesive.

### EXPLANATIONS OF LETTERS OR NUMERALS

1 Capsule endoscope
2 Casing
2a Cylindrical body
2b, 2c Optical dome
3a to 3d, 6a to 6d Light-emitting element
4, 7 Optical unit
4a, 4b, 7a, 7b Lens
4c, 7c Aperture unit
4d, 7d Lens frame
5, 8 Solid-state imaging device
9a Wireless unit
9b Antenna
10 Control unit
11a Magnetic switch
11b, 11c Capacitor
11d Power supply IC
12a, 12b Battery
13a, 13b Contact spring
14, 15 Positioning unit
14a, 15a Plate-like portion
14b, 15b Protrusion
16, 17, 36 Load receiving unit
18a, 18b Power supply board
19a, 19f Illuminating board
19b, 19e Imaging board
19c Control board
19d Wireless board
20 Series of circuit boards
20a, 20b Series of flexible boards
21 Adhesive
22 Metal wire
23 Sealing resin
25 Anisotropic conductive adhesive
27 Metal bump
28 Insulating adhesive
100 Pressure receiving jig
A1 to A5 Extending part
CL Central axis
E1, E2 Mounting area
H1, H2 Opening part

### BEST MODE(S) FOR CARRYING OUT THE INVENTION

Exemplary embodiments of a capsule medical device and a method of manufacturing a capsule medical device according to the present invention will be explained below in detail with reference to the accompanying drawings. A capsule endoscope introduced into a subject and having an imaging function for capturing an in-vivo image, which is an example of in-vivo information of the subject, and a wireless communication function for wirelessly transmitting the captured in-vivo image is explained as an example of the capsule medical device manufactured by the manufacturing method of the present invention. However, the present invention is not limited to the embodiments.

### (Embodiment)

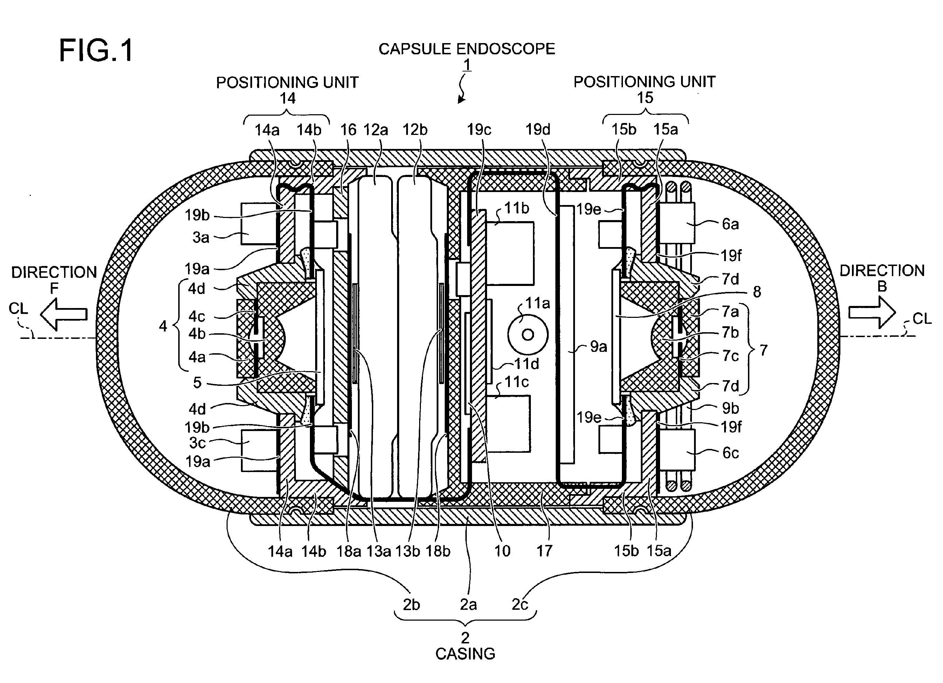

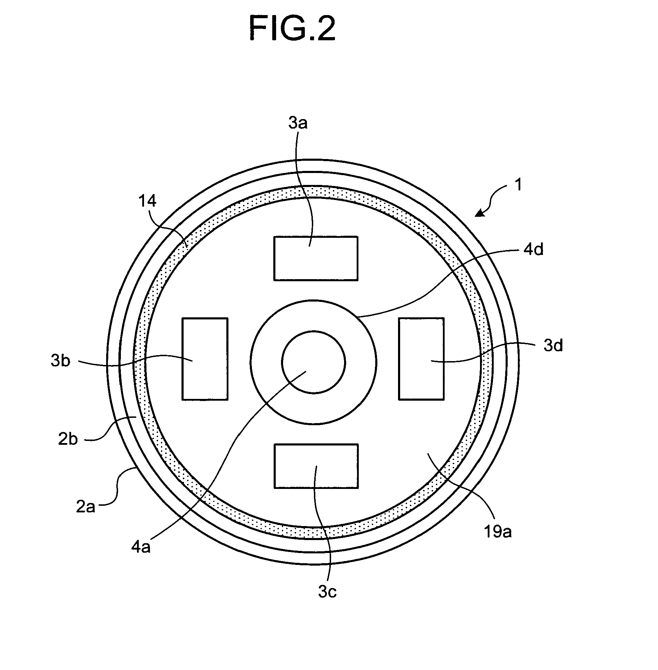

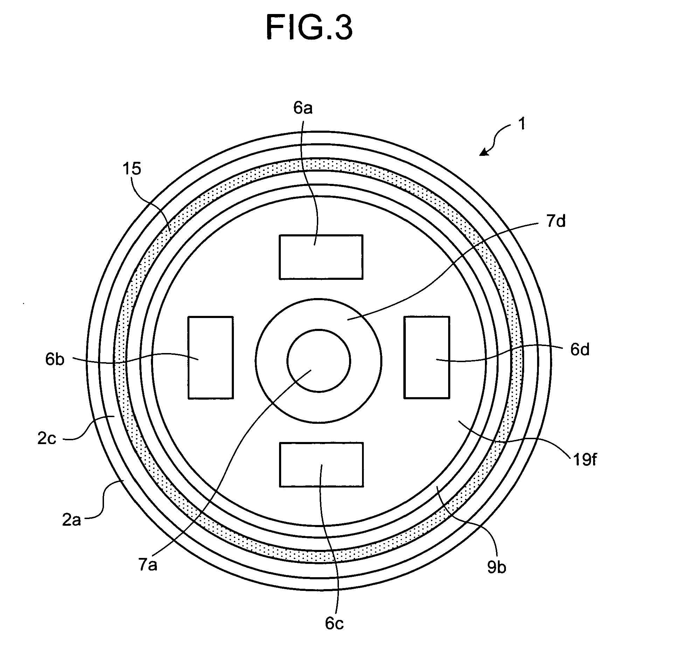

FIG. 1 is a schematic longitudinal cross section of a configuration example of a capsule endoscope according to an embodiment of the present invention. FIG. 2 is a schematic diagram for exemplifying an internal structure of the capsule endoscope as viewed over an optical dome from a direction F shown in FIG. 1. FIG. 3 is a schematic diagram for exemplifying the internal structure of the capsule endoscope as viewed over the optical dome from a direction B shown in FIG. 1.

As shown in FIG. 1, a capsule endoscope 1 according to the embodiment of the present invention is a binocular-lens capsule endoscope that captures an in-vivo image on a direction F side (forward side) and an in-vivo image on a direction B side (back side). The capsule endoscope 1 includes a capsule casing 2 formed in a size introduceable into internal organs of a subject, and has an imaging function for capturing an in-vivo image on the direction F side, an imaging function for capturing an in-vivo image on the direction B side, and a wireless communication function for wirelessly transmitting in-vivo images captured by these imaging functions to the outside.

Specifically, as shown in FIGS. 1 to 3, the capsule endoscope 1 includes, in the casing 2, an illuminating board 19a including a plurality of light-emitting elements 3a to 3d mounted thereon to illuminate the inside of the subject on the direction F side; an optical unit 4 that forms images of inside the subject illuminated by the light-emitting elements 3a to 3d; and an imaging board 19b including a solid-state imaging device 5 mounted thereon to capture the images of inside the subject formed by the optical unit 4 (that is, the in-vivo image on the direction F side). The capsule endoscope 1 also includes, in the casing 2, an illuminating board 19f including a plurality of light-emitting elements 6a to 6d mounted thereon to illuminate the inside of the subject on the direction B side; an optical unit 7 that forms images of inside the subject illuminated by the light-emitting elements 6a to 6d; and an imaging board 19e including a solid-state imaging device 8 mounted thereon to capture the images of inside the subject formed by the optical unit 7 (that is, the in-vivo image on the direction B side). Further, the capsule endoscope 1 includes, in the casing 2, a wireless board 19d having a wireless unit 9a mounted thereon to wirelessly transmit respective in-vivo images captured by the solid-state imaging devices 5 and 8 to the outside via an antenna 9b, and a control board 19c having a control unit 10 mounted thereon to control the imaging function and the wireless communication function.

The capsule endoscope 1 includes, in the casing 2, a power supply system for supplying electric power to the light-emitting elements 3a to 3d and 6a to 6d, the solid-state imaging devices 5 and 8, the wireless unit 9a, and the control unit 10, that is, various circuit parts such as a magnetic switch 11a; batteries 12a and 12b; power supply boards 18a and 18b; and contact springs 13a and 13b that connect the batteries 12a and 12b with the power supply boards 18a and 18b so that electrical conduction therebetween is established. Further, the capsule endoscope 1 also includes, in the casing 2, a positioning unit 14 that determines respective relative positions of the light-emitting elements 3a to 3d and the optical unit 4 with respect to an optical dome 2b forming the forward end of the casing 2; a positioning unit 15 that determines respective relative positions of the light-emitting elements 6a to 6d and the optical unit 7 with respect to an optical dome 2c forming the backward end of the casing 2; a load receiving unit 16 that receives an elastic force of the contact spring 13a to fix the positioning unit 14 with respect to the optical dome 2b; and a load receiving unit 17 that receives an elastic force of the contact spring 13b to fix the positioning unit 15 with respect to the optical dome 2c.

The casing 2 is a capsule casing having a size easily introduceable into the internal organs of the subject, and is realized by fitting the optical domes 2b and 2c to both opening ends of a cylindrical body 2a having a cylindrical structure. The cylindrical body 2a has an outer diameter larger than that of the optical domes 2b and 2c, so that the optical domes 2b and 2c can be fitted to an inner circumference near the both opening ends. A step that abuts against the end face of the optical domes 2b and 2c at the time of fitting the optical domes 2b and 2c is formed on the inner circumference near the both opening ends of the cylindrical body 2a. The relative positions of the optical domes 2b and 2c with respect to the cylindrical body 2a are determined by abutting the respective end faces of the optical domes 2b and 2c against the step of the cylindrical body 2a.

The optical domes 2b and 2c are optically transparent dome members formed in a substantially uniform thickness. A depression is formed on an outer circumference near the opening end of each of the optical domes 2b and 2c. The depressions engage with protrusions provided on the inner circumference near the opening ends of the cylindrical body 2a. The optical dome 2b is fitted to the inner circumference near the opening end on the forward side (the direction F side shown in FIG. 1) of the cylindrical body 2a, and is attached to the forward-side opening end of the cylindrical body 2a by locking the protrusion on the inner circumference of the cylindrical body 2a in the depression of the optical dome 2b. In this case, the end face of the optical dome 2b is in a state of being abutted against the step on the inner circumference of the cylindrical body 2a. The optical dome 2b forms a part of the capsule casing 2 (specifically, a forward end). Meanwhile, the optical domes 2c is fitted to the inner circumference near the opening end on the back side (the direction B side shown in FIG. 1) of the cylindrical body 2a, and is attached to the back-side opening end of the cylindrical body 2a by locking the protrusion on the inner circumference of the cylindrical body 2a in the depression of the optical dome 2c. In this case, the end face of the optical dome 2c is in a state of being abutted against the step on the inner circumference of the cylindrical body 2a. The optical dome 2c forms a part of the capsule casing 2 (specifically, a backward end). As shown in FIG. 1, the casing 2 including the cylindrical body 2a and the optical domes 2b and 2c liquid-tightly accommodates the respective components of the capsule endoscope 1.

The light-emitting elements 3a to 3d function as an illuminating unit that illuminates the inside of the subject positioned on the direction F side. Specifically, each of the light-emitting elements 3a to 3d is a light-emitting element such as an LED, and is mounted on the illuminating board 19a, which is a flexible board formed in a substantially disk shape. In this case, as shown in FIGS. 1 and 2, the light-emitting elements 3a to 3d are mounted on the illuminating board 19a to surround a lens frame 4d (described later) of the optical unit 4 inserted into an opening part of the illuminating board 19a. The light-emitting elements 3a to 3d emit predetermined illumination light (for example, white light), to illuminate the inside of the subject on the direction F side over the forward-side optical dome 2b.

The number of the light-emitting elements to be mounted on the illuminating board 19a is not specifically limited to four, and can be one or more, so long as the light-emitting element can emit the illumination light with an amount of light sufficient for illuminating the inside of the subject on the direction F side. As exemplified in the light-emitting elements 3a to 3d, when a plurality of light-emitting elements are mounted on the illuminating board 19a, it is desired that the light-emitting elements are mounted thereon at rotationally symmetric positions centering on an optical axis of the optical unit 4 inserted into the opening part of the illuminating board 19a.

The optical unit 4 condenses reflected light from the inside of the subject on the direction F side illuminated by the light-emitting elements 3a to 3d, and forms images of inside the subject on the direction F side. The optical unit 4 is realized by lenses 4a and 4b formed by, for example, injection molding of glass or plastic, an aperture unit 4c arranged between the lenses 4a and 4b, and the lens frame 4d that holds the lenses 4a and 4b and the aperture unit 4c.

The lenses 4a and 4b condense the reflected light from the inside of the subject on the direction F side illuminated by the light-emitting elements 3a to 3d, and forms images of inside the subject on the direction F side on a light receiving surface of the solid-state imaging device 5. The aperture unit 4c narrows down (adjusts) brightness of the reflected light condensed by the lenses 4a and 4b to suitable brightness. The lens frame 4d has a cylindrical structure with the both ends being opened, and holds the lenses 4a and 4b and the aperture unit 4c in a cylindrical portion. The lens frame 4d is fitted and fixed to a through hole in a plate-like portion 14a (described later) of the positioning unit 14, with the lens frame 4d being inserted into an opening part formed in the illuminating board 19a. In this case, an upper end (an opening end on the lens 4a side) and a body of the lens frame 4d are protruded on the illuminating board 19a side, and a lower end thereof is locked to a peripheral portion of the through hole in the plate-like portion 14a. The lens frame 4d fixed to the plate-like portion 14a of the positioning unit 14 holds the lenses 4a and 4b at predetermined positions determined by the positioning unit 14 (that is, suitable relative positions with respect to the optical dome 2b). The lenses 4a and 4b can match a longitudinal central axis CL of the casing 2 with the optical axis.

The lens 4b held by the lens frame 4d has legs as shown in FIG. 1, and determines positional relation between the lens 4b and the solid-state imaging device 5 in an optical axis direction by abutting the legs against a device surface on a light receiving side of the solid-state imaging device 5. Thus, in a manner in which the legs of the lens 4b abut against the device surface on the light receiving side of the solid-state imaging device 5, a clearance is formed between the lower end of the lens frame 4d and the imaging board 19b. A predetermined adhesive is filled in the clearance, and the lower end of the lens frame 4d and the imaging board 19b are bonded to each other by the adhesive. The adhesive and the lens frame 4d block unnecessary light from entering into the lenses 4a and 4b and the light receiving surface of the solid-state imaging device 5.

The solid-state imaging device 5 is a CCD, CMOS, or the like having the light receiving surface, and functions as an imaging unit that captures images of inside the subject on the direction F side illuminated by the light-emitting elements 3a to 3d. Specifically, the solid-state imaging device 5 is mounted (for example, flip-chip mounted) on the imaging board 19b, which is the flexible board formed in a substantially disk shape, so that the lens 4b faces the light receiving surface via an opening part of the imaging board 19b. In this case, the solid-state imaging device 5 causes the device surface thereof on the light receiving side to abut against the legs of the lens 4b, and is fixed and arranged with respect to the optical unit 4 by adhesion between the imaging board 19b and the lower end of the lens frame 4d, while maintaining the abutting state with respect to the legs of the lens 4b. The solid-state imaging device 5 receives the reflected light from the inside of the subject condensed by the lenses 4a and 4b via the light receiving surface, and captures images of inside the subject formed on the light receiving surface by the lenses 4a and 4b (that is, an in-vivo image on the direction F side).

The light-emitting elements 6a to 6d function as an illuminating unit that illuminates the inside of the subject positioned on the direction B side. Specifically, each of the light-emitting elements 6a to 6d is a light-emitting element such as an LED, and is mounted on the illuminating board 19f, which is a flexible board formed in a substantially disk shape. In this case, as shown in FIGS. 1 and 3, the light-emitting elements 6a to 6d are mounted on the illuminating board 19f to surround a lens frame 7d (described later) of the optical unit 7 inserted into an opening part of the illuminating board 19f. The light-emitting elements 6a to 6d emit predetermined illumination light (for example, white light), to illuminate the inside of the subject on the direction B side over the back-side optical dome 2c.

The number of the light-emitting elements to be mounted on the illuminating board 19f is not specifically limited to four, and can be one or more, so long as the light-emitting element can emit the illumination light with an amount of light sufficient for illuminating the inside of the subject on the direction B side. As exemplified in the light-emitting elements 6a to 6d, when a plurality of light-emitting elements are mounted on the illuminating board 19f, it is desired that the light-emitting elements are mounted thereon at rotationally symmetric positions centering on an optical axis of the optical unit 7 inserted into the opening part of the illuminating board 19f.

The optical unit 7 condenses the reflected light from the inside of the subject on the direction B side illuminated by the light-emitting elements 6a to 6d and forms images of inside the subject on the direction B side. The optical unit 7 is realized by lenses 7a and 7b formed by, for example, injection molding of glass or plastic, an aperture unit 7c arranged between the lenses 7a and 7b, and the lens frame 7d that holds the lenses 7a and 7b and the aperture unit 7c.

The lenses 7a and 7b condense the reflected light from the inside of the subject on the direction B side illuminated by the light-emitting elements 6a to 6d, and forms the images of inside the subject on the direction B side on a light receiving surface of the solid-state imaging device 8. The aperture unit 7c narrows down (adjusts) brightness of the reflected light condensed by the lenses 7a and 7b to suitable brightness. The lens frame 7d has a cylindrical structure with the both ends being opened, and holds the lenses 7a and 7b and the aperture unit 7c in a cylindrical portion. The lens frame 7d is fitted and fixed to a through hole in a plate-like portion 15a (described later) of the positioning unit 15, with the lens frame 7d being inserted into the opening part formed in the illuminating board 19f. In this case, an upper end (an opening end on the lens 7a side) and a body of the lens frame 7d are protruded on the illuminating board 19f side, and a lower end thereof is locked to a peripheral portion of the through hole in the plate-like portion 15a. The lens frame 7d fixed to the plate-like portion 15a of the positioning unit 15 holds the lenses 7a and 7b at predetermined positions determined by the positioning unit 15 (that is, suitable relative positions with respect to the optical dome 2c). The lenses 7a and 7b can match the longitudinal central axis CL of the casing 2 with the optical axis.

The lens 7b held by the lens frame 7d has legs (see FIG. 1) as the lens 4b of the optical unit 4, and determines positional relation between the lens 7b and the solid-state imaging device 8 in the optical axis direction by abutting the legs against a device surface on a light receiving side of the solid-state imaging device 8. Thus, in a manner in which the legs of the lens 7b abut against the device surface on the light receiving side of the solid-state imaging device 8, a clearance is formed between the lower end of the lens frame 7d and the imaging board 19e. A predetermined adhesive is filled in the clearance, and the lower end of the lens frame 7d and the imaging board 19e are bonded to each other by the adhesive. The adhesive and the lens frame 7d block unnecessary light from entering into the lenses 7a and 7b and the light receiving surface of the solid-state imaging device 8.

The solid-state imaging device 8 is a CCD, CMOS, or the like having the light receiving surface, and functions as an imaging unit that captures images of inside the subject on the direction B side illuminated by the light-emitting elements 6a to 6d. Specifically, the solid-state imaging device 8 is mounted (for example, flip-chip mounted) on the imaging board 19e, which is a flexible board formed in a substantially disk shape, so that the lens 7b faces the light receiving surface via an opening part of the imaging board 19e. In this case, the solid-state imaging device 8 causes the device surface thereof on the light receiving side to abut against the legs of the lens 7b, and is fixed and arranged with respect to the optical unit 7 by adhesion between the imaging board 19e and the lower end of the lens frame 7d, while maintaining the abutting state with respect to the legs of the lens 7b. The solid-state imaging device 8 receives the reflected light from the inside of the subject condensed by the lenses 7a and 7b via the light receiving surface, and captures images of inside the subject formed on the light receiving surface by the lenses 7a and 7b (that is, an in-vivo image on the direction B side).

The wireless unit 9a and the antenna 9b realize the wireless communication function for wirelessly transmitting each of in-vivo images on the direction F or the direction B side captured by the solid-state imaging devices 5 and 8 to the outside. Specifically, the wireless unit 9a is mounted on the wireless board 19d, which is the flexible board formed in a substantially disk shape, and is arranged in the casing 2, facing the imaging board 19e having the solid-state imaging device 8 mounted thereon. As shown in FIGS. 1 and 3, the antenna 9b is fixed and arranged on the illuminating board 19f fixed on the surface of the plate-like portion 15a of the positioning unit 15, and is connected to the wireless unit 9a via the wireless board 19d and the illuminating board 19f. In this case, the antenna 9b is fixed and arranged on an outer edge of the illuminating board 19f facing the optical dome 2c at the backward end and outside of the light-emitting elements 6a to 6d.

When having acquired an image signal including the in-vivo image on the direction F side captured by the solid-state imaging device 5, the wireless unit 9a performs modulation or the like with respect to the acquired image signal each time, to generate a wireless signal including the in-vivo image on the direction F side, and transmits the generated wireless signal to the outside via the antenna 9b. Meanwhile, when having acquired an image signal including the in-vivo image on the direction B side captured by the solid-state imaging device 8, the wireless unit 9a performs modulation or the like with respect to the acquired image signal each time, to generate a wireless signal including the in-vivo image on the direction B side, and transmits the generated wireless signal to the outside via the antenna 9b. The wireless unit 9a alternately generates the wireless signal including the in-vivo image on the direction F side and the wireless signal including the in-vivo image on the direction B side under control of the control unit 10, and alternately transmits the generated wireless signals.

The control unit 10 is a processor such as a DSP, and is arranged approximately at the center of the casing 2 in a state mounted on the control board 19c, which is a rigid board formed in a substantially disk shape. The control unit 10 is electrically connected to the illuminating boards 19a and 19f, the imaging boards 19b and 19e, and the wireless board 19d via the control board 19c and the flexible board. The control unit 10 controls: the light-emitting elements 3a to 3d mounted on the illuminating board 19a; the light-emitting elements 6a to 6d mounted on the illuminating board 19f; the solid-state imaging devices 5 and 8 mounted on the imaging boards 19b and 19e, respectively; and the wireless unit 9a mounted on the wireless board 19d. Specifically, the control unit 10 controls operation timing of the light-emitting elements 3a to 3d and the solid-state imaging device 5 so that the solid-state imaging device 5 captures the in-vivo image on the direction F side for each predetermined time period, synchronously with a light emitting operation of the light-emitting elements 3a to 3d. Likewise, the control unit 10 controls the operation timing of the light-emitting elements 6a to 6d and the solid-state imaging device 8 so that the solid-state imaging device 8 captures the in-vivo image on the direction B side for each predetermined time period, synchronously with the light emitting operation of the light-emitting elements 6a to 6d. The control unit 10 also controls the wireless unit 9a to wirelessly transmit the in-vivo image on the direction F side and the in-vivo image on the direction B side alternately. The control unit 10 includes various parameters involved with image processing such as white balance, and has an image processing function for sequentially generating the image signal including the in-vivo image on the direction F side captured by the solid-state imaging device 5 and the image signal including the in-vivo image on the direction B side captured by the solid-state imaging device 8.

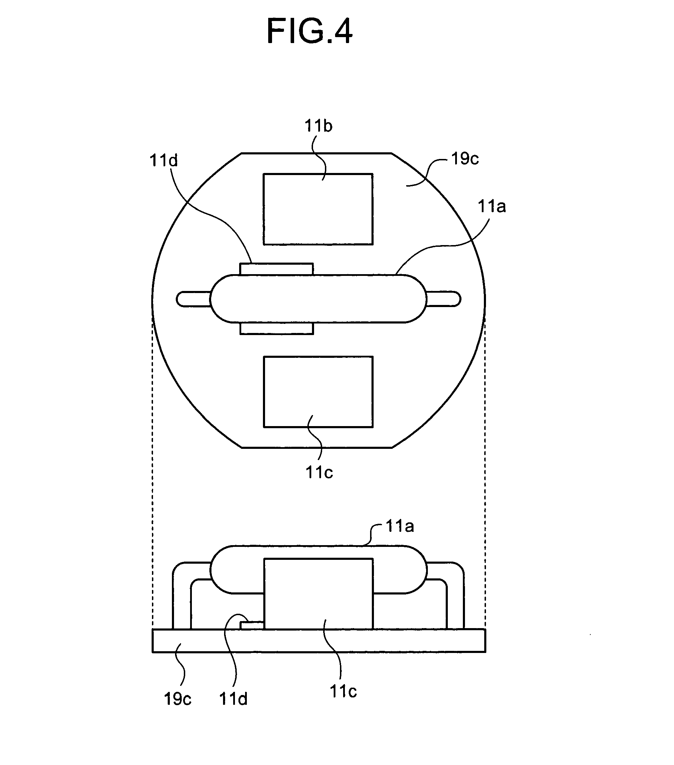

Meanwhile, on the control board 19c, circuit components of the power supply system, that is, various circuit components such as the magnetic switch 11a are mounted on a board surface on the opposite side of the board surface where the control unit 10 is mounted. FIG. 4 is a schematic diagram for exemplifying a state where the circuit components of the power supply system are mounted on the control board 19c. As shown in FIGS. 1 and 4, for example, the magnetic switch 11a, capacitors 11b and 11c, and a power supply IC 11d are mounted on one board surface of the control board 19c, as the circuit components of the power supply system. In this case, the capacitors 11b and 11c and the power supply IC 11d are surface-mounted on the control board 19c, and the magnetic switch 11a is mounted on the control board 19c, spanning over the power supply IC 11d using a lead extending from the both ends of the magnetic switch 11a. The magnetic switch 11a switches ON/OFF by applying an external magnetic field in a predetermined direction. In a case of ON state, the magnetic switch 11a starts to supply power to the light-emitting elements 3a to 3d and 6a to 6d, the solid-state imaging devices 5 and 8, the wireless unit 9a, and the control unit 10 from the batteries 12a and 12b, and in a case of OFF state, the magnetic switch 11a stops supplying power from the batteries 12a and 12b. Meanwhile, the power supply IC 11d has a power supply control function for controlling the power supply to the respective components via the magnetic switch 11a.

The batteries 12a and 12b generate power for operating the light-emitting elements 3a to 3d and 6a to 6d, the solid-state imaging devices 5 and 8, the wireless unit 9a, and the control unit 10. Specifically, the batteries 12a and 12b are button batteries such as a silver oxide battery, and as shown in FIG. 1, are arranged between the load receiving units 16 and 17 and held by an end of the positioning unit 14 and an end of the load receiving unit 17. The power supply boards 18a and 18b electrically connected to the control board 19c via the flexible board or the like are provided on surfaces of the load receiving units 16 and 17, respectively, which are facing the batteries 12a and 12b, respectively. The conductive contact springs 13a and 13b are provided on the power supply boards 18a and 18b, respectively. The batteries 12a and 12b arranged between the load receiving units 16 and 17 are held by the end of the positioning unit 14 and the end of the load receiving unit 17 in a manner in which the contact springs 13a and 13b are contracted, and are electrically connected to the circuit components (the magnetic switch 11a, the capacitors 11b and 11c, and the power supply IC 11d) of the power supply system on the control board 19c via the contracted contact springs 13a and 13b and the power supply boards 18a and 18b. The number of batteries arranged in the casing 2 is not particularly limited two, so long as the required power can be supplied.

The illuminating board 19a including the light-emitting elements 3a to 3d mounted thereon and the optical unit 4 are fixed and arranged in the positioning unit 14, and the positioning unit 14 is fitted and fixed to an inner circumference of the forward-side optical dome 2b. The positioning unit 14 fitted and fixed to the inner circumference of the optical dome 2b fixes the positional relation of the optical dome 2b, the light-emitting elements 3a to 3d, and the optical unit 4, and determines suitable relative positions of the light-emitting elements 3a to 3d and the optical unit 4 with respect to the optical dome 2b. The positioning unit 14 includes the plate-like portion 14a fitted to the inner circumference of the optical dome 2b and a protrusion 14b for fixing the plate-like portion 14a at a predetermined position on the inner circumference of the optical dome 2b.

The plate-like portion 14a is a substantially disk plate member having an outer diameter matched with an inner diameter of the optical dome 2b, and has an outer circumference fitted to the inner circumference of the optical dome 2b. The illuminating board 19a and the optical unit 4 are fixed and arranged on the plate-like portion 14a. Specifically, the plate-like portion 14a fixes and arranges the illuminating board 19a on a surface facing the optical dome 2b, when being fitted to the inner circumference of the optical dome 2b. The plate-like portion 14a has a through hole that communicates with an opening part formed in the illuminating board 19a substantially at a center thereof, and the lens frame 4d of the optical unit 4 is inserted into and fixed (for example, fitted and fixed) in the through hole. The lens frame 4d inserted into and fixed in the through hole of the plate-like portion 14a protrudes the upper end and the body thereof on the illuminating board 19a side in a state of being inserted into the opening part of the illuminating board 19a. The plate-like portion 14a fixes the positional relation between the lens frame 4d and the light-emitting elements 3a to 3d so that the respective upper ends of the light-emitting elements 3a to 3d are positioned at a lower position than the upper end of the lens frame 4d.

The protrusion 14b protrudes from the plate-like portion 14a, and is locked to the opening end of the optical dome 2b to fix the plate-like portion 14a on the inner circumference of the optical dome 2b. Specifically, the protrusion 14b is integrally formed with the plate-like portion 14a, and protrudes from a back of the surface of the plate-like portion 14a, on which the illuminating board 19a is fixed and arranged. The protrusion 14b has a cylindrical structure having an outer diameter matched with the inner diameter of the optical dome 2b (that is, outer diameter same as that of the plate-like portion 14a), and includes a flange that engages with the opening end of the optical dome 2b at the opening end of the cylindrical structure. The protrusion 14b having such a structure is fitted to the inner circumference of the optical dome 2b together with the plate-like portion 14a, and locks the flange to the opening end of the optical dome 2b. Accordingly, the protrusion 14b fixes the plate-like portion 14a at the predetermined position on the inner circumference of the optical dome 2b.

The illuminating board 19f including the light-emitting elements 6a to 6d mounted thereon and the optical unit 4 are fixed and arranged in the positioning unit 15, and the positioning unit 15 is fitted and fixed to an inner circumference of the backward-side optical dome 2c. The positioning unit 15 fitted and fixed to the inner circumference of the optical dome 2c fixes the positional relation of the optical dome 2c, the light-emitting elements 6a to 6d, and the optical unit 7, and determines suitable relative positions of the light-emitting elements 6a to 6d and the optical unit 7 with respect to the optical dome 2c. The positioning unit 15 includes the plate-like portion 15a fitted to the inner circumference of the optical dome 2c and a protrusion 15b for fixing the plate-like portion 15a at a predetermined position on the inner circumference of the optical dome 2c.

The plate-like portion 15a is a substantially disk plate member having an outer diameter matched with an inner diameter of the optical dome 2c, and has an outer circumference fitted to the inner circumference of the optical dome 2c. The illuminating board 19f and the optical unit 7 are fixed and arranged on the plate-like portion 15a. Specifically, the plate-like portion 15a fixes and arranges the illuminating board 19f on a surface facing the optical dome 2c, when being fitted to the inner circumference of the optical dome 2c. The plate-like portion 15a has a through hole that communicates with an opening part formed in the illuminating board 19f substantially at a center thereof, and the lens frame 7d of the optical unit 7 is inserted into and fixed (for example, fitted and fixed) in the through hole. The lens frame 7d inserted into and fixed in the through hole of the plate-like portion 15a protrudes the upper end and the body thereof on the illuminating board 19f side in a state of being inserted into the opening part of the illuminating board 19f. The plate-like portion 15a fixes the positional relation between the lens frame 7d and the light-emitting elements 6a to 6d so that the respective upper ends of the light-emitting elements 6a to 6d are positioned at a lower position than the upper end of the lens frame 7d.

The protrusion 15b protrudes from the plate-like portion 15a, and is locked to the opening end of the optical dome 2c to fix the plate-like portion 15a on the inner circumference of the optical dome 2c. Specifically, the protrusion 15b is integrally formed with the plate-like portion 15a, and protrudes from a back of the surface of the plate-like portion 15a, on which the illuminating board 19f is fixed and arranged. The protrusion 15b has a cylindrical structure having an outer diameter matched with the inner diameter of the optical dome 2c (that is, outer diameter same as that of the plate-like portion 15a), and includes a flange that engages with the opening end of the optical dome 2c at the opening end of the cylindrical structure. The protrusion 15b having such a structure is fitted to the inner circumference of the optical dome 2c together with the plate-like portion 15a, and locks the flange to the opening end of the optical dome 2c. Accordingly, the protrusion 15b fixes the plate-like portion 15a at the predetermined position on the inner circumference of the optical dome 2c.

Upon reception of the elastic force (spring force) of the contact spring 13a, the load receiving unit 16 presses and fixes the positioning unit 15 to the opening end of the optical dome 2c by the elastic force. Specifically, the load receiving unit 16 is a plate member having a substantially disk shape that engages the outer edge thereof with a step formed on an inner circumference of the protrusion 15b of the positioning unit 14, and includes the power supply board 18a and the contact spring 13a on the surface facing the battery 12a. The load receiving unit 16 presses and fixes the flange of the protrusion 14b to the opening end of the optical dome 2b by the elastic force of the contact spring 13a, upon reception of the elastic force of the contact spring 13a generated with contraction of the contact spring 13a. In this case, the load receiving unit 16 fits and fixes the plate-like portion 14a integral with the protrusion 14b at the predetermined position on the inner circumference of the optical dome 2b by pressing and fixing the protrusion 14b to the opening end of the optical dome 2b.

As shown in FIG. 1, the through hole for avoiding a contact with the circuit components such as the capacitor mounted on the imaging board 19b is provided in the load receiving unit 16. When the load receiving unit 16 is engaged with the step on the inner circumference of the protrusion 14b, the load receiving unit 16 and the positioning unit 14 form a space, as shown in FIG. 1, sufficient for arranging the solid-state imaging device 5 abutting against the legs of the lens 4b and the imaging board 19b fixed with respect to the lower part of the lens frame 4d.

Upon reception of the elastic force (spring force) of the contact spring 13b, the load receiving unit 17 presses and fixes the positioning unit 15 to the opening end of the optical dome 2c by the elastic force. Specifically, the load receiving unit 17 is a member having a cylindrical structure having a slightly smaller outer diameter than an inner diameter of the cylindrical body 2a of the casing 2, and including a plate-like portion facing the battery 12b at one opening end of the cylindrical structure.

The cylindrical structure of the load receiving unit 17 functions as a spacer that forms a predetermined space in the casing 2, and engages the other opening end with the opening end (flange) of the protrusion 15b of the positioning unit 15. In this case, as shown in FIG. 1, the cylindrical structure of the load receiving unit 17 and the positioning unit 15 forms a space sufficient for arranging the control board 19c including the control unit 10 and the circuit components such as the magnetic switch 11a mounted thereon, the wireless board 19d including the wireless unit 9a mounted thereon, the solid-state imaging device 8 abutting against the legs of the lens 7b, and the imaging board 19e fixed with respect to the lower part of the lens frame 7d.

Meanwhile, the plate-like portion of the load receiving unit 17 is integrally formed with the cylindrical structure of the load receiving unit 17 at one opening end thereof, and as shown in FIG. 1, includes the power supply board 18b and the contact spring 13b on the surface facing the battery 12b. The plate-like portion of the load receiving unit 17 has a through hole for preventing a contact with the circuit components such as the capacitor mounted on the control board 19c, arranged in the space formed by the cylindrical structure of the load receiving unit 17. The plate-like portion of the load receiving unit 17 receives the elastic force of the contact spring 13b generated with contraction of the contact spring 13b, and presses the cylindrical structure of the load receiving unit 17 to the opening end of the protrusion 15b of the positioning unit 15 by the elastic force of the contact spring 13b.

The load receiving unit 17 having the cylindrical structure and the plate-like portion presses and fixes the flange of the protrusion 15b to the opening end of the optical dome 2c by the elastic force of the contact spring 13b. In this case, the load receiving unit 17 presses and fixes the protrusion 15b to the opening end of the optical dome 2c, thereby fitting and fixing the plate-like portion 15a integral with the protrusion 15b to a predetermined position on the inner circumference of the optical dome 2c.

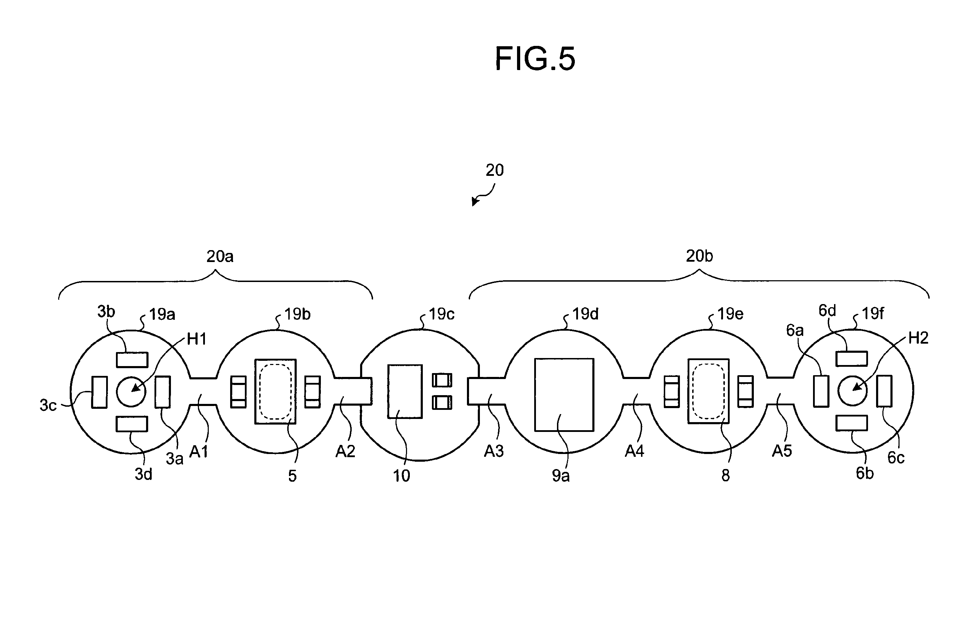

A series of circuit boards (specifically, the illuminating boards 19a and 19f, the imaging boards 19b and 19e, the control board 19c, and the wireless board 19d) arranged in the casing 2 of the capsule endoscope 1 is explained next. FIG. 5 is a schematic diagram for exemplifying a state where the series of circuit boards folded and arranged in the casing 2 of the capsule endoscope 1 is developed. Each board surface of the flexible board or the rigid board shown in FIG. 5 is defined as a board surface at the front (front board surface), and a back face of the front board surface shown in FIG. 5 is defined as a board surface at the back (back board surface).

As shown in FIG. 5, a series of circuit boards 20 arranged in the casing 2 of the capsule endoscope 1 is achieved by electrically connecting a series of flexible boards 20a connecting the illuminating board 19a and the imaging board 19b, the control board 19c as the rigid board, and a series of flexible boards 20b connecting the wireless board 19d, the imaging board 19e, and the illuminating board 19f.

The illuminating board 19a is flexible board having a substantially disk shape, on which a circuit for realizing an illuminating function for illuminating the subject on the direction F side of the capsule endoscope 1 is formed. The plurality of light-emitting elements 3a to 3d are mounted on the front board surface of the illuminating board 19a, and an opening part H1 for inserting the lens frame 4d of the optical unit 4 having the lens 4b, in a manner in which the legs thereof abut against the solid-state imaging device 5, is formed at the center of the board surface of the illuminating board 19a surrounded by the light-emitting elements 3a to 3d. The illuminating board 19a is electrically connected to the imaging board 19b via an extending part A1, which is a flexible board extending from an outer edge.

The imaging board 19b is a flexible board having a substantially disk shape, on which a circuit for realizing the imaging function for capturing the in-vivo image on the direction F side is formed. The solid-state imaging device 5 is flip-chip mounted on the front board surface of the imaging board 19b, and the circuit components such as the capacitor are mounted thereon as required. As shown by a dotted line in FIG. 5, in the imaging board 19b, there is formed an opening part for the reflected light from inside of the subject on the direction F side to enter into a light-receiving surface of the flip-chip mounted solid-state imaging device 5. Although not specifically shown in FIG. 5, the lower end of the lens frame 4d of the optical unit 4 abutting against the legs of the lens 4b is fixed on the light-receiving side device surface of the solid-state imaging device 5 via the opening part of the imaging board 19b, as shown in FIG. 1. The imaging board 19b is electrically connected to the control board 19c via an extending part A2, which is a flexible board extending from the outer edge.

The control board 19c is a rigid board having a substantially disk shape, on which a circuit necessary for the power supply system such as the magnetic switch 11a and the control unit 10 is formed. The control unit 10 is mounted on the front board surface of the control board 19c, and the circuit components such as the capacitor are mounted thereon as required. Meanwhile, as shown in FIG. 4, the magnetic switch 11a, the capacitors 11b and 11c, and the power supply IC 11d, which are the circuit components of the power supply system, are mounted on the back board surface of the control board 19c. The control board 19c is electrically connected to the wireless board 19d via an extending part A3, which is a flexible board extending from the outer edge of the wireless board 19d. Although not specifically shown in FIG. 5, the control board 19c is electrically connected to the power supply boards 18a and 18b via the flexible board or the like (not shown).

The wireless board 19d is a flexible board having a substantially disk shape, on which a circuit for realizing the wireless communication function for wirelessly transmitting the in-vivo image on the direction F side and the in-vivo image on the direction B side sequentially to the outside is formed. The wireless unit 9a is mounted on the front board surface of the wireless board 19d. Although not particularly shown in FIG. 5, the wireless board 19d is electrically connected to the antenna 9b fixed and arranged on the outer edge of the illuminating board 19f, as shown in FIGS. 1 and 3. The wireless board 19d is electrically connected to the imaging board 19e via an extending part A4, which is a flexible board extending from the outer edge.

The imaging board 19e is a flexible board having a substantially disk shape, on which a circuit for realizing the imaging function for capturing the in-vivo image on the direction B side is formed. The solid-state imaging device 8 is flip-chip mounted on the front board surface of the imaging board 19e, and the circuit components such as the capacitor are mounted as required. As shown by a dotted line in FIG. 5, in the imaging board 19e, there is formed an opening part for the reflected light from inside of the subject on the direction F side to enter into a light-receiving surface of the flip-chip mounted solid-state imaging device 8. Although not specifically shown in FIG. 5, the lower end of the lens frame 7d of the optical unit 7 abutting against the legs of the lens 7b is fixed on the light-receiving side device surface of the solid-state imaging device 8 via the opening part of the imaging board 19e, as shown in FIG. 1. The imaging board 19e is electrically connected to the illuminating board 19f via an extending part A5, which is a flexible board extending from the outer edge.

The illuminating board 19f is a flexible board having a substantially disk shape, on which a circuit that realizes the illuminating function for illuminating the subject on the direction B side of the capsule endoscope 1 is formed. The light-emitting elements 6a to 6d described above are mounted on the front board surface of the illuminating board 19f, and an opening part H2 for inserting the lens frame 7d of the optical unit 7 having the lens 7b in a manner in which the legs abut against the solid-state imaging device 8 is formed at the center of the board surface of the illuminating board 19f surrounded by the light-emitting elements 6a to 6d.

The series of flexible boards 20a is a circuit board group having the illuminating board 19a and the imaging board 19b, and is formed as an integrally formed flexible board obtained by connecting the illuminating board 19a with the imaging board 19b. The series of flexible boards 20a has a series of circuit board structure connecting the imaging board 19b having the extending part A2 for connecting to the control board 19c extending from the outer edge and the illuminating board 19a with each other via the extending part A1. On the other hand, the series of flexible boards 20b is a circuit board group having the wireless board 19d, the imaging board 19e, and the illuminating board 19f, and is formed as an integrally formed flexible board obtained by connecting the wireless board 19d, the imaging board 19e, and the illuminating board 19f. The series of flexible boards 20b has a series of circuit board structure connecting the wireless board 19d having the extending part A3 for connecting to the control board 19c extending from the outer edge and the imaging board 19e with each other via the extending part A4, and a series of board structure connecting the imaging board 19e and the illuminating board 19f with each other via the extending part A5. The series of circuit board 20 arranged in the casing 2 of the capsule endoscope 1 is realized by connecting the series of flexible boards 20a and 20b with the control board 19c via the extending parts A2 and A3.

A manufacturing method of the capsule endoscope 1 according to the embodiment of the present invention is explained next. The capsule endoscope 1 is manufactured by preparing the series of circuit boards 20 having the necessary functional components mounted thereon (see FIG. 5), preparing a functional unit by combining the manufactured series of circuit boards 20, the positioning units 14 and 15, the load receiving units 16 and 17, and the batteries 12a and 12b, and arranging the manufactured functional unit in the casing 2.

Specifically, the series of circuit boards 20 shown in FIG. 5 is manufactured by connecting the series of flexible boards 20a on which the necessary functional components such as the light-emitting elements 3a to 3d and the solid-state imaging device 5 are mounted, and the series of flexible boards 20b on which the necessary functional components such as the light-emitting elements 6a to 6d and the solid-state imaging device 8 are mounted to the control board 19c in a good product state, having the necessary functional components such as the control unit 10 mounted thereon. The good product state referred to here is a state where the respective functional components mounted on the respective circuit boards normally operate. Details of a manufacturing method of the series of circuit boards 20 are described later.

The functional unit of the capsule endoscope 1 is then manufactured by combining the series of circuit boards 20 manufactured as described above, the positioning units 14 and 15, the load receiving units 16 and 17, and the batteries 12a and 12b. The functional unit is the one excluding the casing 2 of the capsule endoscope 1 shown in FIG. 1 (that is, a unit arranged in the casing 2).

In the functional unit, the lens frame 4d of the optical unit 4 mounted on the imaging board 19b is fitted and fixed in a through hole formed in the plate-like portion 14a of the positioning unit 14. An adhesive or a double-sided tape is applied or attached to one surface of the plate-like portion 14a (a surface facing the optical dome 2b) as a bonding member, and the illuminating board 19a is fixed to the plate-like portion 14a by the bonding member, with the lens frame 4d being inserted into the opening part H1. The outer edge of the load receiving unit 16 is engaged with the protrusion 14b of the positioning unit 14, to which the illuminating board 19a and the imaging board 19b are fitted. In this case, the load receiving unit 16 is fitted to the protrusion 14b in a manner in which the power supply board 18a and the contact spring 13a are arranged on the backward side of the surface facing the solid-state imaging device 5 of the imaging board 19b.

Meanwhile, the lens frame 7d of the optical unit 7 mounted on the imaging board 19e is fitted and fixed in the through hole formed in the plate-like portion 15a of the positioning unit 15. The adhesive or double-sided tape is applied or attached to one surface of the plate-like portion 15a (a surface facing the optical dome 2c) as a bonding member, and the illuminating board 19f is fixed to the plate-like portion 15a by the bonding member, with the lens frame 7d being inserted into the opening part H2. An end of the cylindrical structure of the load receiving unit 17 is engaged with the protrusion 15b of the positioning unit 15, to which the illuminating board 19f and the imaging board 19e are fitted. In this case, the load receiving unit 17 is fitted to the protrusion 15b in a state where the control board 19c and the wireless board 19d are arranged in the space formed by the cylindrical structure, and the power supply board 18b and the contact spring 13b can be arranged to face the power supply board 18a and the contact spring 13a of the load receiving unit 16.

Further, the batteries 12a and 12b are arranged between the load receiving units 16 and 17, in which the power supply board 18b and the contact spring 13b face the power supply board 18a and the contact spring 13a. In this case, the batteries 12a and 12b are held by the protrusion 14b of the positioning unit 14 an the end of the load receiving unit 17, with a positive pole and a negative pole thereof coming in contact with each other. The batteries 12a and 12b cause the contact springs 13a and 13b to contract, and are electrically connected to the power supply boards 18a and 18b via the contact springs 13a and 13b.

The functional unit of the capsule endoscope 1 is manufactured as described above. The series of circuit boards 20 incorporated in the functional unit is folded in a predetermined manner. In this case, the respective circuit boards in the series of circuit boards 20 (that is, the illuminating board 19a and the imaging board 19b in the series of flexible boards 20a, the illuminating board 19f, the imaging board 19e, and the wireless board 19d in the series of flexible boards 20b, and the control board 19c) are arranged substantially parallel to each other and facing each other. Specifically, as shown in FIG. 1, the back board surface of the illuminating board 19a and the back board surface of the imaging board 19b face each other via the plate-like portion 14a of the positioning unit 14, and the front board surface of the imaging board 19b and the front board surface of the control board 19c face each other via the load receiving units 16 and 17 and the batteries 12a and 12b. Further, the back board surface of the control board 19c and the back board surface of the wireless board 19d face each other, the front board surface of the wireless board 19d and the front board surface of the imaging board 19e face each other, and the back board surface of the imaging board 19e and the back board surface of the illuminating board 19f face each other via the plate-like portion 15a of the positioning unit 15. The extending part A1 is inserted into a notch (not shown) formed in the positioning unit 14, and the extending part A2 is inserted into notches (not shown) formed in the protrusion 14b of the positioning unit 14 and the load receiving unit 17. The extending part A3 is inserted into a notch (not shown) formed in the cylindrical structure of the load receiving unit 17, the extending part A4 is inserted into notches (not shown) formed in the opening end of the load receiving unit 17 and the protrusion 15b of the positioning unit 14, and the extending part A5 is inserted into a notch (not shown) formed in the positioning unit 15.

Thereafter, the functional unit including the folded series of circuit boards 20 is arranged in the capsule casing 2. That is, the functional unit is inserted into the cylindrical body 2a, and the optical domes 2b and 2c are fitted to respective inner circumferences near the both opening ends of the cylindrical body 2a, which houses the functional unit. In this case, as shown in FIG. 1, the optical domes 2b and 2c are fitted to the respective inner circumferences near the both opening ends of the cylindrical body 2a and fixed by the adhesive or the like, thereby completing the capsule endoscope 1 as shown in FIG. 1.

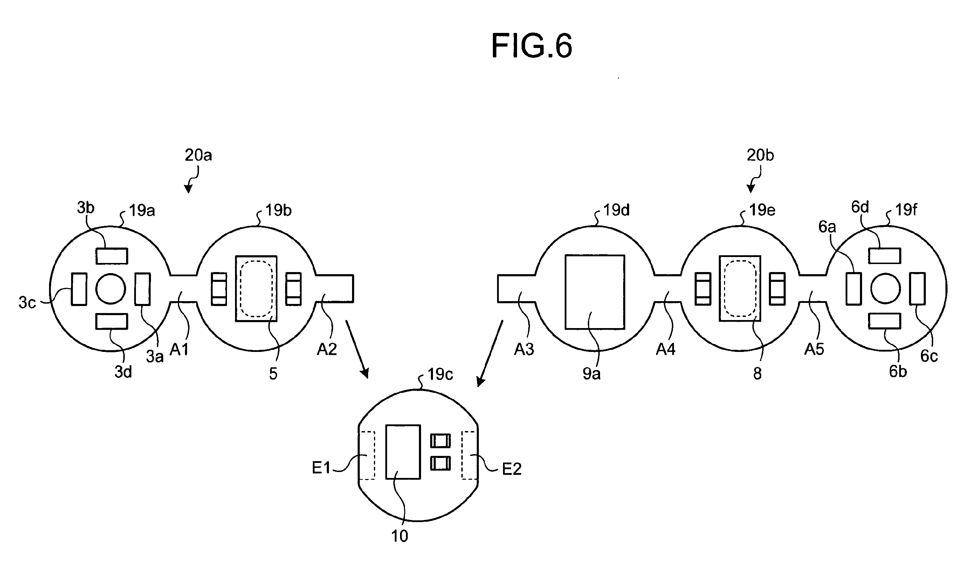

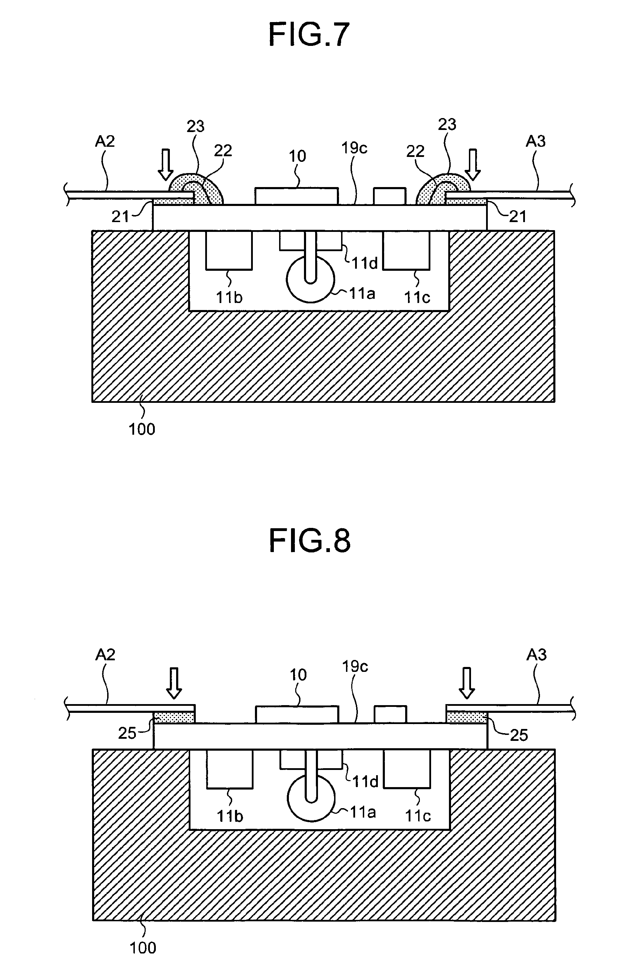

A manufacturing method of the series of circuit boards 20 incorporated in the functional unit of the capsule endoscope 1 is explained next in detail. FIG. 6 is a schematic diagram for explaining the manufacturing method of the series of circuit boards 20 incorporated in the functional unit of the capsule endoscope 1. FIG. 7 is a schematic diagram for exemplifying a state where the series of flexible boards 20a and 20b are connected to the control board 19c. The manufacturing method of the series of circuit boards 20 is explained with reference to FIGS. 6 and 7.

First, the series of flexible boards 20a including the illuminating board 19a and the imaging board 19b, the series of flexible boards 20b including the illuminating board 19f, the imaging board 19e, and the wireless board 19d, and the control board 19c as the rigid board are formed separately (a board forming step). At the board forming step, the series of flexible boards 20a, which is an integrally formed flexible board connecting the illuminating board 19a and the imaging board 19b with each other via the extending part A1 is formed. Further, the series of flexible boards 20b, which is an integrally formed flexible board connecting the wireless board 19d, the imaging board 19e, and the illuminating board 19f via the extending parts A4 and A5, and a separate body from the series of flexible boards 20a, is formed. The control board 19c, which is a separate body from the series of flexible boards 20a and 20b is formed as well.

Required functional components are then mounted on the series of flexible boards 20a and 20b and the control board 19c formed separately at the board forming step (a mounting step). Specifically, at the mounting step, the plurality of light-emitting elements 3a to 3d are mounted on the illuminating board 19a, and the solid-state imaging device 5 and the circuit components such as the capacitor are mounted on the imaging board 19b in the series of flexible boards 20a. In this case, the light-emitting elements 3a to 3d, the solid-state imaging device 5, and the like are mounted on the same side surfaces of the respective boards of the series of flexible boards 20a. That is, the light-emitting elements 3a to 3d are mounted on the front board surface of the illuminating board 19a, and the solid-state imaging device 5, the capacitor, and the like are mounted on the front board surface of the imaging board 19b.

At the mounting step, the plurality of light-emitting elements 6a to 6d and the antenna 9b (see FIG. 1) are mounted on the illuminating board 19f, the solid-state imaging device 8 and the circuit components such as the capacitor are mounted on the imaging board 19e, and the wireless unit 9a is mounted on the wireless board 19d in the series of flexible boards 20b. In this case, the light-emitting elements 6a to 6d, the solid-state imaging device 8, the wireless unit 9a, and the like are mounted on the same side surfaces of the respective boards of the series of flexible boards 20b. That is, the light-emitting elements 6a to 6d and the antenna 9b are mounted on the front board surface of the illuminating board 19f, the solid-state imaging device 8, the capacitor, and the like are mounted on the front board surface of the imaging board 19e, and the wireless unit 9a is mounted on the front board surface of the wireless board 19d.

Further, at the mounting step, required functional components such as the control unit 10 are mounted on the control board 19c. Specifically, the control unit 10 and the circuit components such as the capacitor are mounted on the front board surface of the control board 19c, and the circuit components of the power supply system (the magnetic switch 11a, the capacitors 11b and 11c, and the power supply IC 11d) are mounted on the back board surface of the control board 19c. In this case, mounting areas E1 and E2 for connecting the respective extending parts A2 and A3 of the series of flexible boards 20a and 20b are ensured on the front board surface of the control board 19c. Further, an unpopulated area (not shown) for placing the control board 19c on a pressure receiving jig 100 shown in FIG. 7 is ensured on the back board surface of the control board 19c.

Subsequently, it is verified whether the respective functional components mounted on the series of flexible boards 20a and 20b and the control board 19c operate normally (a verifying step). At the verifying step, a light-emitting operation of the light-emitting elements 3a to 3d mounted on the illuminating board 19a and an imaging operation of the solid-state imaging device 5 mounted on the imaging board 19b in the series of flexible boards 20a are verified, to determine whether each of the light-emitting elements 3a to 3d and the solid-state imaging device 5 operates normally. When the light-emitting elements 3a to 3d and the solid-state imaging device 5 operate normally, it is determined that the series of flexible boards 20a is in a good product state.

Further, at the verifying step, the light-emitting operation of the light-emitting elements 6a to 6d mounted on the illuminating board 19f, the imaging operation of the solid-state imaging device 8 mounted on the imaging board 19e, and a wireless communication operation of the wireless unit 9a mounted on the wireless board 19d in the series of flexible boards 20b are verified, to determine whether each of the light-emitting elements 6a to 6d, the solid-state imaging device 8, and the wireless unit 9a operate normally. When the light-emitting elements 6a to 6d, the solid-state imaging device 8, and the wireless unit 9a operate normally, it is determined that the series of flexible boards 20b is in a good product state.

Further, at the verifying step, respective operations of the control unit 10 and the magnetic switch 11a mounted on the control board 19c are verified, to determine whether the control unit 10 and the magnetic switch 11a operate normally. When the control unit 10 and the magnetic switch 11a operate normally, it is determined that the control board 19c is in a good product state.

When the series of flexible boards 20a is not in a good product state (a failed state where at least one of the light-emitting elements 3a to 3d and the solid-state imaging device 5 does not operate normally due to defective assembly or the like), the series of flexible boards 20a is replaced by another series of flexible boards 20a, which is in a good product state. Likewise, when the series of flexible boards 20b is not in a good product state (a failed state where at least one of the light-emitting elements 6a to 6d, the solid-state imaging device 8, and the wireless unit 9a does not operate normally due to defective assembly or the like), the series of flexible boards 20b is replaced by another series of flexible boards 20b, which is in a good product state.

The series of flexible boards 20a and 20b determined to be in a good product state at the verifying step are then connected to the control board 19c (a board connecting step). At the board connecting step, as shown in FIG. 6, the series of flexible boards 20a in a good product state is connected to the mounting area E1 of the control board 19c in a good product state, and the series of flexible boards 20b in a good product state is connected to the mounting area E2 of the control board 19c.

Specifically, as shown in FIG. 7, the control board 19c in a good product state is placed on the pressure receiving jig 100, in a manner in which the unpopulated area on the back board surface thereof are brought into contact with the pressure receiving jig 100. The pressure receiving jig 100 receives pressure applied to each board at the time of connecting the control board 19c with the series of flexible boards 20a and 20b in a good product state, and supports the back board surface (specifically, the unpopulated area) of the control board 19c. The pressure receiving jig 100 is provided with a depression for avoiding a contact with the circuit components (the magnetic switch 11a, the capacitors 11b and 11c, and the power supply IC 11d) on the back board surface of the control board 19c at the time of placing the control board 19c.

An adhesive 21 for bonding the series of flexible boards 20a and 20b is applied to the mounting areas E1 and E2 of the control board 19c placed on the pressure receiving jig 100, and the respective extending parts A2 and A3 of the series of flexible boards 20a and 20b in a good product state are pressed thereto via the adhesive 21. The adhesive 21 to which the extending parts A2 and A3 are pressed is heated and cured while being pressurized, to bond the extending parts A2 and A3 to the mounting areas E1 and E2 of the control board 19c, respectively. Thereafter, respective terminals of the control board 19c and respective terminals of the extending parts A2 and A3 are electrically connected with each other by bonding of metal wires 22 including gold or aluminum, and the metal wires 22 each connecting the terminals with each other is covered with a sealing resin 23. In this case, the respective metal wires 22 are protected from an external force by the sealing resin 23.

As described above, board-to-board connection for electrically and physically connecting the control board 19c and the series of flexible boards 20a and 20b in a good product state via the extending parts A2 and A3 is achieved. According to the board-to-board connection between the control board 19c and the series of flexible boards 20a and 20b, a series of circuit boards 20 having a series of board structures is manufactured, as shown in FIG. 5, in which the series of flexible boards 20a in a good product state, the control board 19c in a good product state, and the series of flexible boards 20b in a good product state are connected.

Thereafter, the optical units 4 and 7 are fitted to the imaging boards 19b and 19e, respectively, of the series of circuit boards 20. In this case, the optical unit 4 is fitted to the back board surface of the imaging board 19b in a manner in which the solid-state imaging device 5 on the imaging board 19b abut against the legs of the lens 4b. The optical unit 7 is fitted to the back board surface of the imaging board 19e in a manner in which the solid-state imaging device 8 on the imaging board 19e abut against the legs of the lens 7b.

The lens frame 4d of the optical unit 4 is a separate body with respect to the positioning unit 14, and fixed on the back board surface of the imaging board 19b before being fitted and fixed in the through hole of the positioning unit 14 (specifically, the plate-like portion 14a) as shown in FIG. 1. Therefore, a working space required for applying the adhesive to a clearance between the imaging board 19b and the lower end of the lens frame 4d can be ensured sufficiently, and the lens frame 4d can be easily fixed to the imaging board 19b by the adhesive. The same applies to the lens frame 7d fitted to the back board surface of the imaging board 19e.

As in the conventional capsule medical device, when the functional components are mounted on an integrally formed rigid flexible board in a manner in which a plurality of rigid flexible boards such as the illuminating board and the imaging board being connected via the flexible board, if a failure such as defective assembly occurs in one of the functional components, even if the remaining functional components are in a good product state, all the functional components including the functional components in a good product state mounted on the rigid flexible board need to be discarded together with a part of the functional components in the failed state, and the rigid flexible board in a good product state needs to be manufactured again. Specifically, when the light-emitting elements 3a to 3d and the solid-state imaging device 5 on the forward side (the direction F side shown in FIG. 1) and the light-emitting elements 6a to 6d and the solid-state imaging device 8 on the backward side (the direction B side shown in FIG. 1) are mounted on the rigid flexible board, if defective assembly occurs in, for example, the solid-state imaging device 5, even if the remaining light-emitting elements 3a to 3d and 6a to 6d, and the solid-state imaging device 8 are in a good product state, the entire rigid flexible board including the light-emitting elements 3a to 3d and 6a to 6d, and the solid-state imaging device 8 in a good product state need to be discarded together with the solid-state imaging device 5 in the failed state. Therefore, in many cases, the functional components in a good product state are discarded wastefully, and as a result, causing a decrease in a manufacturing yield of the capsule medical device.

On the other hand, according to the manufacturing method of the capsule endoscope 1 of the embodiment of the present invention, the light-emitting elements 3a to 3d on the forward side are mounted on the illuminating board 19a, and the solid-state imaging device 5 on the forward side is mounted on the imaging board 19b in the series of flexible boards 20a, while the light-emitting elements 6a to 6d on the backward side are mounted on the illuminating board 19f, and the solid-state imaging device 8 on the backward side is mounted on the imaging board 19e in the series of flexible boards 20b that is a separate body from the series of flexible boards 20a. Therefore, a failure such as defective assembly occurs in the functional components (the light-emitting elements 3a to 3d or the solid-state imaging device 5) mounted on, for example, the series of flexible boards 20a, only the series of flexible boards 20a in a failed state needs only to be replaced, and hence, the various functional components mounted on the remaining control board 19c and the series of flexible boards 20b in a good product state are not discarded wastefully. Likewise, even if a failure such as defective assembly occurs in the wireless unit 9a or the antenna 9b mounted on the series of flexible boards 20b (that is, components other than the functional components associated with capturing of in-vivo images), only the series of flexible boards 20b in the failed state needs only to be replaced by the one in a good product state. Therefore, various functional components mounted on the remaining control board 19c and the series of flexible boards 20a in a good product state are not discarded wastefully. As a result, the manufacturing yield of the capsule endoscope 1 can be increased, and the manufacturing cost of the capsule endoscope 1 can be reduced.

According to the manufacturing method of the capsule medical device of the embodiment of the present invention, one or more functional components are mounted on each of a first circuit board group (for example, the series of flexible boards 20a) and a second circuit board group (for example, the series of flexible boards 20b) formed separately from each other, and the first circuit board group and the second circuit board group, on which required functional components are mounted, are connected to the control board, thereby manufacturing a series of circuit boards having the required functional components. Therefore, when a failure such as defective assembly occurs in the first circuit board group, the second circuit board group, or the control board, only the circuit board in the failed state can be replaced with the functional component in a good product state, without wastefully discarding the remaining functional components which are in a good product state. Accordingly, the first circuit board group in a good product state, the second circuit board group in a good product state, and the control board in a good product state can be board-to-board connected efficiently. As a result, even if a part of the functional components mounted on the circuit board is in the failed state, the capsule medical device can be manufactured without wastefully discarding the remaining functional components in a good product state. According to the manufacturing method of the capsule medical device of the present invention, the manufacturing yield of the capsule medical device can be increased, and the manufacturing cost of the capsule medical device can be reduced.

According to the manufacturing method of the capsule medical device of the embodiment of the present invention, the flexible board is used as the circuit board such as the illuminating board, the imaging board, and the wireless board. Accordingly, downsizing and weight saving of the capsule medical device can be facilitated and the board cost can be reduced, as compared to the conventional manufacturing method of the capsule medical device using the rigid board as the circuit board.

Further, according to the manufacturing method of the capsule medical device of the embodiment of the present invention, for component mounting surfaces of the first and second circuit board groups (the series of flexible boards 20a and 20b), on which various functional components are mounted, the same side surfaces (for example, the front board surfaces) of the respective boards are used, and the various functional components such as the light-emitting elements, the solid-state imaging devices, and the wireless unit are mounted on the same side surfaces of the respective boards of the first and second circuit board groups. Accordingly, the required various functional components can be easily mounted on the first and second circuit board groups.

In the embodiment of the present invention, the extending parts A2 and A3 are bonded to the mounting areas E1 and E2, respectively, of the control board 19c with the thermosetting adhesive 21, and the respective terminals of the extending parts A2 and A3 and the respective terminals of the control board 19c are electrically connected with each other by using the metal wires 22, so that the series of flexible boards 20a and 20b and the control board 19c are board-to-board connected. However, the present invention is not limited thereto, and the series of flexible boards 20a and 20b and the control board 19c may be board-to-board connected by using an anisotropic conductive adhesive. In this case, as shown in FIG. 8, an anisotropic conductive adhesive 25 is arranged in (applied to) the mounting areas E1 and E2 of the control board 19c, so that the extending part A2 of the series of flexible board 20a and the extending part A3 of the series of flexible board 20b are bonded to the mounting areas E1 and E2, respectively, of the control board 19c with the anisotropic conductive adhesive 25, and the respective terminals of the extending parts A2 and A3 and the respective terminals of the mounting areas E1 and E2 are electrically connected with each other.

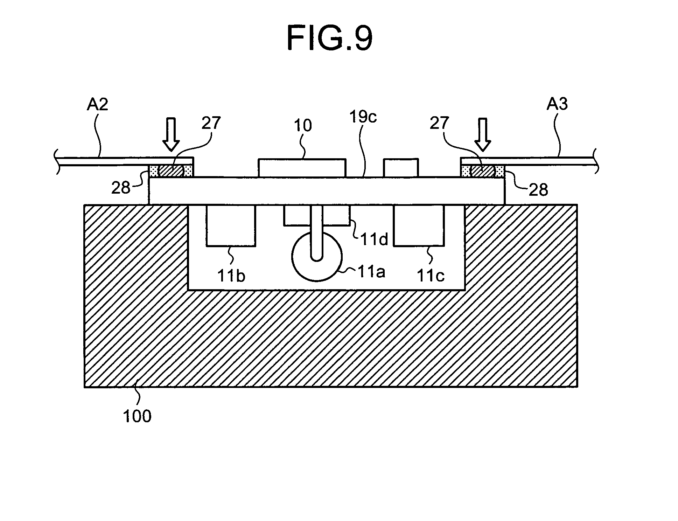

The series of flexible boards 20a and 20b and the control board 19c may be board-to-board connected by using not only the anisotropic conductive adhesive, but also by using a metal bump including solder or gold and an insulating adhesive. In this case, as shown in FIG. 9, the respective terminals of the mounting areas E1 and E2 of the control board 19c and the respective terminals of the extending parts A2 and A3 are electrically connected with each other by using metal bumps 27, and an insulating adhesive 28 is filled in gaps between the extending parts A2 and A3 and the control board 19c where the metal bumps 27 are arranged, so that the extending parts A2 and A3 and the mounting areas E1 and E2 of the control board 19c are bonded, respectively, with the insulating adhesive 28.

Further, in the embodiment of the present invention, the series of flexible boards 20a connecting the illuminating board 19a and the imaging board 19b on the forward side and the series of flexible boards 20b connecting the illuminating board 19f, the imaging board 19e, and the wireless board 19d on the backward side are formed separately from each other. However, the present invention is not limited thereto, and the series of flexible boards formed separately needs only to include at least the illuminating board and the imaging board. For example, a series of flexible boards connecting the illuminating board 19a and the imaging board 19b on the forward side, a series of flexible boards connecting the illuminating board 19f and the imaging board 19e on the backward side, and the wireless board 19d may be formed separately from each other. In this case, when a failure such as defective assembly occurs in the wireless board 19d, the wireless board 19d in a failed state may be replaced with one in a good product state, without wastefully discarding the series of flexible boards.

In the embodiment of the present invention, as the capsule medical device introduced into the subject, a capsule endoscope having the imaging function and the wireless communication function, which acquires in-vivo images as an example of the in-vivo information is explained. However, the present invention is not limited thereto, and the capsule medical device can be a capsule pH measuring device that measures pH information in a living body as the in-vivo information, a capsule drug-administering device having a function of spraying or injecting a drug into the living body, or a capsule sampling device that samples a substance in the living body (tissue of the body) as the in-vivo information.

### INDUSTRIAL APPLICABILITY

As described above, the capsule medical device and the method of manufacturing a capsule medical device according to the present invention are useful for a capsule medical device introduced into a subject, and particularly suitable for a capsule medical device that can be manufactured, even if a part of functional components mounted on the circuit board is in a failed state, without wastefully discarding remaining functional components in a good product state, and a manufacturing method of the capsule medical device.

## Claims

1. A method of manufacturing a capsule medical device, comprising:
a mounting step of mounting one or more functional components on each of a first circuit board group and a second circuit board group, which are separate bodies from each other, and mounting a control unit that controls an operation of the one or more functional components, on a control board that is a separate body from the first circuit board group and the second circuit board group; and
a board connecting step of connecting the first circuit board group and the second circuit board group to the control board.

2. The method of manufacturing a capsule medical device according to claim 1, further comprising:
a verifying step of verifying whether the one or more functional components mounted on the first circuit board group operate normally, verifying whether the one or more functional components mounted on the second circuit board group operate normally, and verifying whether the control unit mounted on the control board operates normally, wherein
at the board connecting step, the first circuit board group in a good product state and the second circuit board group in a good product state having been determined to operate normally at the verifying step are connected to the control board in a good product state having been determined to operate normally at the verifying step.

3. The method of manufacturing a capsule medical device according to claim 1 or 2, wherein at the mounting step, the one or more functional components are mounted on same side surfaces of boards of the first circuit board group, and the one or more functional components are mounted on same side surfaces of boards of the second circuit board group.

4. The method of manufacturing a capsule medical device according to any one of claims 1 to 3, further comprising:
a board forming step of separately forming the first circuit board group, which is an integrally formed flexible circuit board including an illuminating board and an imaging board, the second circuit board group, which is an integrally formed flexible circuit board including at least an illuminating board and an imaging board, and the control board, which is a rigid circuit board, wherein
at the mounting step, an illuminating unit and an imaging unit as functional components for capturing an in-vivo image of inside a subject are mounted on the illuminating board and the imaging board, respectively, in the first circuit board group, and an illuminating unit and an imaging unit as functional components for capturing an in-vivo image in a different direction than the in-vivo image are mounted on the illuminating board and the imaging board, respectively, in the second circuit board group.

5. The method of manufacturing a capsule medical device according to claim 4, wherein
at the board forming step, the second circuit board group, which is a integrally formed flexible circuit board including the illuminating board, the imaging board, and a wireless board, is formed, and
at the mounting step, a wireless unit, which is a functional component for wirelessly transmitting the in-vivo images and the in-vivo images in the different direction to outside, is mounted on the wireless board in the second circuit board group.

6. The method of manufacturing a capsule medical device according to any one of claims 1 to 5, further comprising:
a board arranging step of arranging circuit boards in a series of circuit boards formed of the first circuit board group, the second circuit board group, and the control board connected at the board connecting step, substantially parallel to each other and facing each other; and
an inside-casing arranging step of arranging at least the series of circuit boards inside a capsule casing.

7. A capsule medical device comprising:
a first circuit board group on which one or more functional components are mounted;
a second circuit board group on which one or more functional components are mounted; and
a control board on which a control unit that controls operations of the one or more functional components in the first circuit board group and the one or more functional components in the second circuit board group are mounted, wherein
the first circuit board group, the second circuit board group, and the control board are separate bodies from each other, and
the first circuit board group, the second circuit board group, and the control board are formed as a series of circuit boards obtained by connecting good circuit boards each having been determined to operate normally to each other.

8. The capsule medical device according to claim 7, wherein the one or more functional components mounted on the first circuit board group and the one or more functional components mounted on the second circuit board group include functional components having a same function.

9. The capsule medical device according to claim 8, wherein the same function means an illuminating unit and an imaging unit for capturing an in-vivo image of inside a subject.

10. The capsule medical device according to claim 9, wherein the illuminating unit and the imaging unit mounted on the first circuit board group and the illuminating unit and the imaging unit mounted on the second circuit board group capture in-vivo images in directions different from each other.
